# EUROPEAN PATENT SPECIFICATION

(11) **EP 3 043 655 B1**
(45) Date of publication and mention of the grant of the patent: **22.11.2017**
(21) Application number: 14766652.3
(22) Date of filing: 10.09.2014
(51) Int. Cl.: A23K 50/80, A23K 20/26, A23K 20/24

(54) **COMPOSITION COMPRISING PHOSPHOROUS FOR USE IN PREVENTING DEFORMITIES IN TRIPLOID FISH**
ZUSAMMENSETZUNG MIT PHOSPHORGEHALT ZUR VERWENDUNG BEI DER VERHINDERUNG VON DEFORMIERUNGEN BEI TRIPLOIDEN FISCHEN
COMPOSITION COMPRENANT DU PHOSPHORE À UTILISER POUR LA PRÉVENTION DES MALFORMATIONS CHEZ LE POISSON TRIPLOÏDE

(30) Priority: 11.09.2013 EP 13183966
(43) Date of publication of application: 20.07.2016
(73) Proprietor: Biomar Group, 8000 Aarhus C (DK)
(72) Inventor: WALTON, Jamie, Edinburgh EH11 1SB (GB); CAMPBELL, Patrick, Haddington EH41 4 DF (GB); MIGAUD, Herve, Dunblane Stirlingshire FK15 0FE (GB); TAYLOR, John, Alloa FK10 1LY (GB)
(74) Representative: Plougmann Vingtoft a/s
(86) International application number: PCT/DK2014/050282
(87) International publication number: WO 2015/035999

(56) References cited:
- Heather Anne Burke ET AL: "THE RESPONSE OF TRIPLOID ATLANTIC SALMON (SALMO SALAR) TO VARYING LEVELS OF ENERGY AND PHOSPHORUS A THESIS SUBMITTED IN PARTIAL FULFILLMENT OF THE REQUIREMENTS FOR THE DEGREE OF Master of Science In the Graduate Academic Unit of Biology Co-Supervisors", , 2009, XP055101966, Retrieved from the Internet: URL:http://dspace.hil.unb.ca:8080/bitstrea m/handle/1882/44249/MR80705.pdf?sequence=1 [retrieved on 2014-02-13]
- PER GUNNAR FJELLDAL ET AL: "Inadequate phosphorus nutrition in juvenile Atlantic salmon has a negative effect on long-term bone health", AQUACULTURE, ELSEVIER, AMSTERDAM, NL, vol. 334, 23 December 2011 (2011-12-23), pages 117-123, XP028458736, ISSN: 0044-8486, DOI: 10.1016/J.AQUACULTURE.2011.12.043 [retrieved on 2012-01-04]
- P.G. FJELLDAL ET AL: "Supplementation of dietary minerals during the early seawater phase increase vertebral strength and reduce the prevalence of vertebral deformities in fast-growing under-yearling Atlantic salmon ( Salmo salar L.) smolt", AQUACULTURE NUTRITION, vol. 15, no. 4, August 2009 (2009-08), pages 366-378, XP055102345, ISSN: 1353-5773, DOI: 10.1111/j.1365-2095.2008.00601.x
- FONTAGNE S ET AL: "Effects of dietary phosphorus and calcium level on growth and skeletal development in rainbow trout (Oncorhynchus mykiss) fry", AQUACULTURE, ELSEVIER, AMSTERDAM, NL, vol. 297, no. 1-4, 1 December 2009 (2009-12-01), pages 141-150, XP026719146, ISSN: 0044-8486, DOI: 10.1016/J.AQUACULTURE.2009.09.022 [retrieved on 2009-09-30]

## Description

### Technical field of the invention

The present invention relates to a composition for use in the prevention of deformities in triploid fish. The present invention also relates to methods for the manufacture of a fish feed.

### Background of the invention

The escape and subsequent breeding of farmed e.g. Atlantic salmon with wild fish is considered a threat to the genetic diversity of wild populations. With over two decades of selective breeding for traits of interest, the genetic variation of farmed Atlantic salmon stocks has been reduced. Interbreeding between escapes and wild populations is therefore likely to result in reduced genetic variability of wild stock through genetic introgression. With a total number of farm-raised Atlantic salmon escapes in the North Atlantic estimated at around 2 million per annum this issue is being addressed by the industry through a number of initiatives.

The production of triploid fish, a ploidy manipulation inducing sterility, is so far the only reliable and commercially acceptable technique for the genetic containment of farmed stocks and protection of wild populations. Although triploids are easily produced by these techniques they are generally characterized by poor performance. Triploid Atlantic Salmon have been reported to show lower survival and growth performance.

Despite the potential benefits, there is reluctance to commercially implement triploid production principally due to high prevalence of anatomical deformities which are above commercially and welfare acceptable limits. A variety of pathologies have been reported at hatch and throughout early fry development, while in on growing stages in both fresh and salt water, reported deformities include ocular cataracts, opercular shortening, gill filament deformity syndrome and reduced gill surface area, lower jaw deformity syndrome and vertebral deformities. These vertebral deformities, with 20 different groups characterised (Witten et al., 2009), are of particular interest since they represent a significant economic loss to the industry via reduced growth and survival, increased discards and downgrading at processing. Furthermore, these deformities appear to develop in different regions of the vertebral column depending on the life stage.
Such deformities have also been reported in other types of triploid fish such as rainbow trout.
Clearly, for large scale commercial use of triploids to be viable there is a need to reduce the high occurrence of deformities in triploid fish. FONTAGNE S ET AL. ("Effects of dietary phosphorus and calcium level on growth and skeletal development in rainbow trout (Oncorhynchus mykiss) fry", AQUACULTURE, vol. 297, no. 1-4, December 2009, pages 141-150) discloses that a composition comprising 22g/kg feed prevents the appearance of deformities in diploid rainbow trout. PER GUNNAR FJELLDAL ET AL. ("Inadequate phosphorus nutrition in juvenile Atlantic salmon has a negative effect on long-term bone health",AQUACULTURE, vol. 334, 23 December 2011, pages 117-123) discloses a fish feed comprising 21 g/ kg feed of phosphorus, the source of phosphorus being MCP, said composition being administered to diploid Atlantic salmon juveniles prior to their transfer into seawater.

### Summary of the invention

Thus, an object of the present invention relates to a means preventing deformities in triploid fish.
Thus, one aspect of the invention relates to a composition comprising at least 23g phosphorus per kg composition for use in preventing deformities in triploid fish.
Another aspect of the present invention relates to the use of phosphorous for the manufacture of a medicament comprising at least 23g phosphorus per kg medicament for the prevention of deformities in triploid fish.
A further aspect of the present invention relates to a method of preventing deformities in triploid in fish, said method comprising administering to said fish a composition comprising at least 23g phosphorus per kg composition. Still another aspect not part of the invention is to provide a method for the manufacture of a fish feed, comprising the steps of:
(i) combining a phosphorous sources or a mixture of phosphorous sources with conventional fish feed ingredients,
(ii) forming fish feed pellets comprising said phosphorous source or a mixture of phosphorous sources and said fish feed ingredients and
(iii) obtaining a fish feed comprising phosphorous.

The present invention will now be described in more detail in the following.

### Detailed description of the invention

As already pointed out above there are severe problems connected to the use of triploid fish in the fish farming industry. Some triploid fish (e.g. salmon and trout) show (i) deformities (such as lower jaw defects), (ii) slower growth rate and (iii) higher mortality that their normal (diploid) relatives.

The inventors of the present invention have surprisingly found that a composition comprising phosphorous can be used to prevent deformities in triploid fish.

In particular the inventors of the present invention show that a composition comprising at least 23 g total phosphorous per kg composition can be used to prevent deformities in triploid fish.

Thus, in one embodiment the present invention pertains to a composition comprising at least 23g phosphorus per kg composition for use in preventing deformities in triploid fish.

Thus, in one embodiment the present invention pertains to a composition comprising at least 23 g total phosphorous per kg composition for use in preventing deformities in triploid fish.

In another embodiment the present invention pertains to the use of phosphorous for the manufacture of a medicament comprising at least 23g phosphorus per kg medicament for the prevention of deformities in triploid fish.

In another embodiment the present invention pertains to the use of phosphorous for the manufacture of a medicament comprising at least 23 g total phosphorous per kg medicament for the prevention of deformities in triploid fish.

In a further embodiment the present invention pertains to a method of preventing deformities in triploid in fish, said method comprising administering to said fish a composition comprising at least 23g phosphorus per kg composition. In a further embodiment the present invention pertains to a method of preventing deformities in triploid in fish, said method comprising administering to said fish a composition comprising at least 23 g total phosphorous per kg composition.

It is to be understood that the term "prevention" also covers prophylactic use of the compound, the medicament or method disclosed above.

### Fish

Triploid fish are fish with three sets of chromosomes instead of the normal two.
The extra chromosomes prevent development of viable eggs or sperm so, if the triploid fish escape, they cannot reproduce. Therefore, since triploid fish was introduced in the fish farming industry to alleviate the negative impact of escaped farmed fish on the environment it may be contemplated that the fish is a fish suitable for farming (e.g. a farmed fish).
Thus, in an embodiment of the present invention the triploid fish is a sterile fish.
The triploid fish may be of the family Salmonidae. More specifically the triploid fish may be of the subfamily of Salmoninae.
In an embodiment of the present invention the fish is selected from the group consisting of fish of the genus *Salmo, Oncorhynchus* and *Salvenis.*
In a further embodiment of the present invention the triploid fish of the genus *Salmo* is selected from the group consisting of Atlantic salmon (*Salmo salar*) and Brown trout (*Salmo trutta*).
In an even further embodiment of the present invention the triploid fish of the genus *Oncorhynchus* is selected from the group consisting of Chinook salmon (*Oncorhynchus tshawytsch*), Rainbow trout (*Oncorhynchus mykiss*), Sockeye salmon (*Oncorhynchus nerka*) and Coho salmon (*Oncorhynchus kisutch*).

In a further embodiment of the present invention the triploid fish of the genus *Salvenis* is selected from the group consisting of Arctic charr (*Salvelinus alpinus*), Brook trout (*Salvelinus fontinalis*) and Lake trout (*Salvelinus namaycush*).

It may in particular be preferred that the triploid fish is selected from the group consisting of Atlantic salmon (*Salmo salar*), Brown trout (*Salmo trutta*) and Rainbow trout.

Salmon (diploid and triploid) is produced by mixing the eggs from one female salmon with the milt of one male salmon for fertilization. Once the eggs have hardened, they are transported to a hatchery for incubation. Each egg batch is incubated separately in trays for approximately 5-6 weeks until they reach the eyed stage and will hatch after a total of approximately 9 weeks. Upon hatch, the alevins or sac fry will continue to develop using the nutrients in their yolk sacs. When their yolk sacs are almost consumed, the young fry are ready to feed. It is from this first feeding phase that the inventors surprisingly found that the diets comprising at least 20g total phosphorous per kg composition and preferably at least 22 g total phosphorous per kg composition can be used to prevent deformities in triploid salmon.

Once the young fry are onto feed they grow quickly over the next 9 to 12 months in freshwater. The fry grow to become fingerlings and then parr and finally undergo developmental changes that prepare them for life in saltwater as smolts. Smolts ready for saltwater are transported from the hatchery to the marine farms, where they are transferred to the net pens to begin the salt water phase of their life cycle. Smolts entered at the sea sites adjust to the saltwater environment and begin feeding. They are fed a high quality diet specially formulated to match the optimal seasonal and life stage requirements of salmon in the wild. Once onto feed, smolts grow quickly in saltwater, reaching a kilogram in size in 6 to 8 months.

Thus, in an embodiment of the present invention the fish is selected from the group consisting of fry, fingerlings, parr and smolts.

Therefore, the composition of the present invention may be advantageously administered to the triploid fish in the period after hatching and until smolt tra nsfer.

Thus, in an embodiment of the present invention the composition of the present invention is administered to the triploid fish in the fresh water (FW) phase.

In a preferred embodiment the composition of the present invention is administered to the triploid fish from hatching and until they attain a size of at least 60g whole body weight.

In the present context the sentence "the period after hatching" is similar to the sentence "the period from first feeding". These sentences are therefore used herein interchangeably.

In another embodiment the composition of the present invention is administered to the fish in the period from first feeding and until the fish attain a size of at least 4 g whole body weight, such as at least 5g whole body weight, e.g. at least 6g whole body weight, such as at least 7g whole body weight, e.g. at least 8g whole body weight, such as at least 9g whole body weight, e.g. at least 10g whole body weight, such as at least 11g whole body weight, e.g. at least 12g whole body weight, such as at least 13g whole body weight, e.g. at least 14g whole body weight, such as at least 15g whole body weight, e.g. at least 20g whole body weight, such as aat least 25g whole body weight, e.g. at least 30g whole body weight, such as at least 35g whole body weight, e.g. at least 40g whole body weight, such as at least 45g whole body weight, e.g. at least 50g whole body weight, such as at least 55g whole body weight, e.g. at least 60g whole body weight, such as in the range from 4g-60g whole body weight, e.g. in the range from 4-60g whole body weight, such as in the range from 5-55g whole body weight, e.g. in the range from 6-50g whole body weight, such as in the range from 7-45g whole body weight, e.g. in the range from 8-40g whole body weight, such as in the range from 9-35g whole body weight, e.g. in the range from 10-30g whole body weight, such as in the range from 11-25g whole body weight, e.g. in the range from 12-20g whole body weight, such as in the range from 13-15g whole body weight,
e.g. in the range from 13-14g whole body weight, preferably in the range from 4-15g whole body weight and even more preberably in the range from 4-6g whole body weight .
In a further embodiment the composition of the present invention is administered to the fish in the period from first feeding and until the fish attain a size of at the most 4 g whole body weight, such as at the most 5g whole body weight, e.g. at the most 6g whole body weight, such as at the most 7g whole body weight, e.g. at the most 8g whole body weight, such as at the most 9g whole body weight, e.g. at the most 10g whole body weight, such as at the most 11g whole body weight, e.g. at the most 12g whole body weight, such as at the most 13g whole body weight, e.g. at the most 14g whole body weight, such as at the most 15g whole body weight, e.g. at the most 20g whole body weight, such as at the most 25g whole body weight, e.g. at the most 30g whole body weight, such as at the most 35g whole body weight, e.g. at the most 40g whole body weight, such as at the most 45g whole body weight, e.g. at the most 50g whole body weight, such as at the most 55g whole body weight, e.g. at the most 60g whole body weight. In a preferred embodiment the compostion of the present invention is administered to the fish from first feeding and until the fish attain a size of at the most 4g whole body weight.
When the fish have attained a size of 4g-15g whole body weight from the first feeding the level of total phosphorous per kg composition may be decreased since it surprisingly appears that the preventive effect of such high amounts of phosphorous (i.e. at least 23 g total phosphorous per kg composition) on deformities in triploid fish is primarily obtained during this specific period of time. After this period phosphorous is still needed to retain the preventive effect and should preferably still be present in the feed at levels below 23g total phosphorous per kg composition at least until the fish are transferred to salt water.

### Deformities

The inventors of the present invention have surprisingly discovered that a composition comprising phosphorous can be used to prevent the occurrence of deformities in triploid fish.
In particular the present inventors have discovered that that a composition comprising at least 23 total phosphorous per kg composition can advantageously be used to prevent the occurrence of deformities in triploid fish.
The inventors speculate that this is due to the fact that triploid fish have a higher requirement for phosphorous during early development than their diploid relatives. This is may be due to cellular differences between diploid and triploids. The deformities especially relevant in terms of triploid fish are deformities selected from the group consisting of vertebral deformities, external deformities and jaw deformities.
Deformities may be categorized as non-severe and severe deformities. In an embodiment of the present invention the deformity is a severe deformity. Non-severe deformities occur at a low/background level in all commercial farming of salmonids. Therefore there is a baseline deformity for diploid fish whereby a low percentage of any population will have a few deformities that are externally visible and can be assessed using X-ray. In triploid freshwater populations however severe deformities have been documented, occurring at a much higher frequency.
A severe deformity may be defined as a deformity wherein 6 or more vertebrae are deformed (> 6 dV), such as 7 or more vertebrae are deformed (> 7 dV), e.g. 8 or more vertebrae are deformed (> 8 dV), such as 9 or more vertebrae are deformed (> 9 dV), e.g. 10 or more vertebrae are deformed (> 10 dV).

### Phosphorous

In the present context the term "phosphorous" in the broadest sense is defined as the chemical element P (atomic number 15) in any form. Phosphorous may thus be present in the composition of the present invention both as part of the existing ingredients and/or phosphorous may be added in any form as further natural and synthetic additives. Thus, any components in the composition stemming from plant or animal sources (i.e. raw materials which may be present in the composition of the present invention) are likely to contain phosphorous, but the amount of phosphorous may be boosted by adding phosphorous containing additives. Phosphorous may preferably be present or added in the form of phosphate salts. Phosphorous may preferably be present in forms that are nontoxic to fish. Phosphorous may preferably be added in forms that minimize impact on the aquatic environment.

Thus, in an embodiment of the present invention phosphorous is selected from the group consisting of Monosodium phosphate (MSP), Monoammonium phosphate (MAP), Monocalcium phosphate (MCP), and phosphorus sources from raw materials.

The amount of phosphorous per kg composition and/or feed in the composition, medicament and/or fish feed may be measured using the following method:
Feed and ingredients are digested with dry ashing.
Approximately 1 g is added to a ceramic dish, ashed at 550 degrees celsius, cooled down to room temp. Then HCl is added to the sample and heated up.
After dilution the sample is analysed using a Vista Pro CCD- with simultaneous ICP-OES.

### Administration and formulation

One preferred administration form of the composition and/or medicament according to the present invention is oral dosing. The dosing is preferably in the form of a feed such as but not limited to a fish feed comprising phosphorous.

Thus, in one embodiment the present invention the composition and/or medicament may be formulated as a fish feed. In a preferred embodiment the composition is a fish feed.

Such fish feed may be selected from the group consisting of pressed fish feed, extruded fish feed and wet semimoist feed. Which of the feed types to choose depends on the fish species, the maturity of the fish and to which environment the feed is to be applied.

In a particular embodiment the fish feed comprises conventional feed ingredients. Thus, the fish feed may comprise phosphorous in combination with conventional feed ingredients.

In a preferred embodiment the conventional feed ingredients may be selected from the group consisting of a carbohydrate source, a protein source, a lipid source, ash, water and any combinations thereof.

Thus, in one embodiment the fish feed of the present invention may comprise ingredients selected from the group consisting of a carbohydrate source, a protein source, a lipid source, ash, water and any combinations thereof.

Typically, the protein source may constitute from 25-55% (w/w) of the composition and/or fish feed, such as from 26-54% (w/w), e.g. from 27-53% (w/w), such as from 28-52% (w/w), e.g. from 27-51% (w/w), such as from 28-50% (w/w), e.g. from 29-49% (w/w), such as from 30-48% (w/w), e.g. from 31-47% (w/w), such as from 32-46% (w/w), e.g. from 33-45% (w/w), such as from 34-44% (w/w), e.g. from 35-43% (w/w), such as from 36-42% (w/w), such as from 37-41% (w/w), e.g. from 38-40% (w/w), such as from 39-40% (w/w), preferably in the range from 30-55% w/w.

The carbohydrate source may constitute from 10-25% (w/w) of the composition and/or fish feed, such as from 11-24% (w/w), e.g. from 12-23% (w/w), such as from 13-24% (w/w), e.g. from 14-23% (w/w), such as from 15-24% (w/w), e.g. from 16-23% (w/w), such as from 17- 22% (w/w), e.g. from 18-21% (w/w), such as from 19-20% (w/w), preferably in the range from 10-15% (w/w).

The lipid source may constitute from 15-40% (w/w) of the composition and/or fish feed, such as from 16-39% (w/w), e.g. from 17-38% (w/w), e.g. 18-37% (w/w), such as from 19-36% (w/w), e.g. from 20-35% (w/w), such as from 21-36% (w/w), e.g. from 22-35% (w/w), such as from 23-36% (w/w), e.g. from 24-35% (w/w), such as from 25-34% (w/w), e.g. from 26-33%, such as from 27-32% (w/w), e.g. from 28-33%, such as from 29-32% (w/w), e.g. from 30-31% preferably in the range from 25-40% (w/w).
Typical protein sources may be fish or blood meal or plant proteins from soybean or other plants. Typical carbohydrate sources may be corn wheat or fababean meal. Typical lipid sources may be fish oil or plant oils from soybean, rapeseed, linseed or others.
Thus, in a particular preferred embodiment the composition, medicament and/or fish feed comprises at least 23g total phosphorous per kg composition medicament or fish feed, e.g. at least 24g total phosphorous per kg composition medicament or fish feed, such as at least 25g total phosphorous per kg composition medicament or fish feed, e.g. at least 26g total phosphorous per kg composition medicament or fish feed, such as at least 27g total phosphorous per kg composition medicament or fish feed, e.g. at least 28g total phosphorous per kg composition medicament or fish feed, such as at least 29g total phosphorous per kg composition medicament or fish feed, e.g. at least 29g total phosphorous per kg composition, medicament or fish feed, at least 23g total phosphorous per kg composition medicament or fish feed and preferably at least 24g total phosphorous per kg composition medicament or fish feed.
In a preferred embodiment the composition, medicament and/or fish comprise in the range from 23g-27g total phosphorous per kg composition medicament or fish feed, e.g. in the range from 24-26g total phosphorous per kg composition, medicament or fish feed, such as in the range from 25g-27g total phosphorous per kg composition medicament or fish feed, e.g. preferably in the range 23g-27g total phosphorous per kg composition and more preferably from 24g-27g total phosphorous per kg composition.
Thus, in a particular preferred embodiment the composition, medicament and/or fish feed comprises at least 2.0% w/w total phosphorous as compared to total composition, medicament or fish feed weight, such as at least 2.1% w/w total phosphorous as compared to total composition, medicament or fish feed weight, e.g. at least 2.2% w/w total phosphorous as compared to total composition medicament or fish feed weight, such as at least 2.3% w/w total phosphorous as compared to total composition, medicament or fish feed weight, e.g. at least 2.4% w/w total phosphorous as compared to total composition, medicament or fish feed weight, such as at least 2.5% w/w total phosphorous as compared to total composition, medicament or fish feed weight, e.g. at least 2.6% w/w total phosphorous as compared to total composition, medicament or fish feed weight, such as at least 2.7% w/w total phosphorous as compared to total composition, medicament or fish feed weight, e.g. at least 2.8% w/w total phosphorous as compared to total composition, medicament or fish feed weight, such as at least 2.9% w/w total phosphorous as compared to total composition, medicament or fish feed weight, e.g. at least 3.0% w/w total phosphorous as compared to total composition, medicament or fish feed weight.
In a preferred embodiment the composition, medicament and/or fish feed comprises in the range from 2.0-3.0% w/w total phosphorous as compared to total composition, medicament or fish feed weight, such as in the range from 2.1-2.9% w/w total phosphorous as compared to total composition, medicament or fish feed weight, e.g. in the range from 2.2-2.8% w/w total phosphorous as compared to total composition, medicament or fish feed weight, such as in the range from 2.3-2.7% w/w total phosphorous as compared to total composition, medicament or fish feed weight, e.g. in the range from 2.4-2.6% w/w total phosphorous as compared to total composition, medicament or fish feed weight, such as in the range from 2.5-2.6% w/w total phosphorous as compared to total composition, medicament or fish feed weight, e.g. preferably in the range from 2.0-2.7% w/w total phosphorous as compared to total composition, medicament or fish feed weight, preferably in the range from 2.2-2.7% w/w total phosphorous as compared to total composition, medicament or fish feed weight, more preferred in the range from 2.3-2.7% w/w total phosphorous as compared to total composition, medicament or fish feed weight and and more preferably in the range from 2.4-2.7% w/w total phosphorous as compared to total composition, medicament or fish feed weight.

In Table 3, it can be clearly seen that the overall severity of deformed vertebrae (≥ 6 dV) was low and decreased significantly with increasing P level. Thus, as can be seen from the occurrence of severe deformities during the fresh water phase in triploid fish is reduced to an acceptable level.

Moreover as can be seen in table 2, the food conversion ratio (FCR*, specific growth rate (SGR) and thermal-unit growth coefficient (TGC) the triploid Atlantic salmon fed the high phosphorous diet remained essentially unchanged - thus, no detrimental effects on performance or growth from the use of this higher phosphorus diet could be measured.

These findings are indeed surprising looking at the prior art where Ketola and Richmond (1994) reported the requirement for rainbow trout for maximal growth and feed conversion to be 11.8 g/kg (1.18%) total dietary P. When the total dietary P was increased to 19.8 g/kg (1.98%) the fish gained significantly less weight, had poorer feed utilization (higher FCR) and lower survival. A similar result was reported by Asgard and Shearer (1997) who reported 10-11 g/kg (1.0-1.1%) dietary phosphorous as the requirement of Atlantic salmon for optimal growth. In fish fed diets with phosphorous levels above 10.4 g/kg the authors reported reduced feed efficiency (calculated as weight gain/feed consumed) and reduced the whole body weight. Moreover Burke et al., 2010 investigated the effects of triploidy on juvenile Atlantic salmon in response to varying levels of dietary phosphorus. 3 levels of phosphorus were fed (low = 7.6 g/kg, med = 9.9 g/kg or high = 13.9 g/kg) to parallel groups of diploid or triploid salmon from 45 g to ∼70g whole body weight in freshwater. The results suggested that 9.9 g/kg total dietary phosphorus met the nutritional requirement of triploid salmon, and that the higher level 13.9 g/kg was detrimental as growth rates declined with increasing dietary phosphorus, and that triploids were less able to cope with high dietary phosphorus compared to diploids.

### Methods for manufacturing of a fish feed

There are numerous recipes for making fish feeds.
The fresh water diets used in this instance were a combination of fish meal, krill meal, soya concentrate, corn gluten, wheat gluten, pea protein, wheat flour, fish oil, a vitamin and mineral premix plus synthetic phosphorus. The raw materials are mixed and ground, before being extruded and dried to form the pellets that are used.
Thus, in a preferred embodiment of the present invention the fish feed comprise one or more ingredients selected from the group consisting of fish meal, krill meal, soya concentrate, corn gluten, wheat gluten, pea protein, wheat flour, fish oil, a vitamin, mineral premix plus synthetic phosphorus and combinations thereof. The fish feed may be a fresh water diet.
In the present context mineral premix is to be understood as a mixture of micro-ingredients including but not limited to vitamins, trace minerals, pigments, medicaments, feed suppliments and other micronutrients in dry form of small particple size blended to homogeneity with addition of sufficient inclusion of a carrier particle, such as but not limited to limestone, calcium carbonate, wheat or other grain flour, with functionality to prevent segregation or separation of the ingredients over time during handling or storage.

Thus, in a further aspect not part of the invention pertains to methods for the manufacture of fish feeds. In general such methods include steps of providing phosphorous (e.g. as a phosphorous sources or a mixture of phosphorous sources) together with any of the conventional feed ingredients or fish feed ingredients defined above.

In an embodiment phosphorous is mixed together with the fish feed ingredients prior to formation of the fish feed pellets.

Thus, in an even further aspect not part of the invention provides a method for manufacturing a fish feed, comprising the steps of:
combining a phosphorous sources or a mixture of phosphorous sources with conventional fish feed ingredients,
forming fish feed pellets comprising said phosphorous source or a mixture of phosphorous sources and said fish feed ingredients and obtaining a fish feed comprising at least 23g total phosphorous per kg feed.

Obviously phosphorous may be from selected from the sources stated above as well as mixture thereof. The same is true in respect of the raw materials - i.e. the conventional fish feed ingredients or fish feed ingredients stated above.
How much phosphorous there needs to be added to the raw material before producing the fish feed or to the fish feed in order to reach at least 23g total phosphorous per kg feed depends on the amount of phosphorous present in the raw materials. Raw materials vary over time, from batch to batch and from producer to producer.
Thus, in a preferred embodiment of the present invention the composition, medicament or fish feed is administered to triploid fish.
Palatability is an important factor in the development of fish feed. Providing the fish with a fish feed comprising at least 23g total phosphorous per kg feed, did not negatively affect feed intake. This is a highly important economically parameter as the feed loss however would lead to a significant loss in the profit gained by the fish farmers. In the present context the terms feed, fish feed and diet are used herin interchangeably.
Clearly, the parts pertaining to the composition, medicament and/or fish feed above are also relevant in terms of this part pertaining to methods for manufacturing of a fish feed.
In the present context the term "combining" is to be understood as the putting together of different conventional feed ingredients and mixing to result in a homogeneous blend of the ingredients. Subsequent grinding or pulverising may also be required to reduce the particle size of the blended ingredients.
In the present context the term "forming" is to be understood as the process of bringing together of the combined feed materials and additives by spherizer, agglomeration, cold press or extrusion to produce or create a pellet or particle or three dimensional matrix regarded as compound feed intended to be fed to fish. After forming of the pellets the water content is reduced by drying then fat or liquid oil can be added to the pellets, typically under vacuum before a cooling stage to result in the final compound feed.

The invention will now be described in further details in the following non-limiting examples.

### Figures

Figure 1:

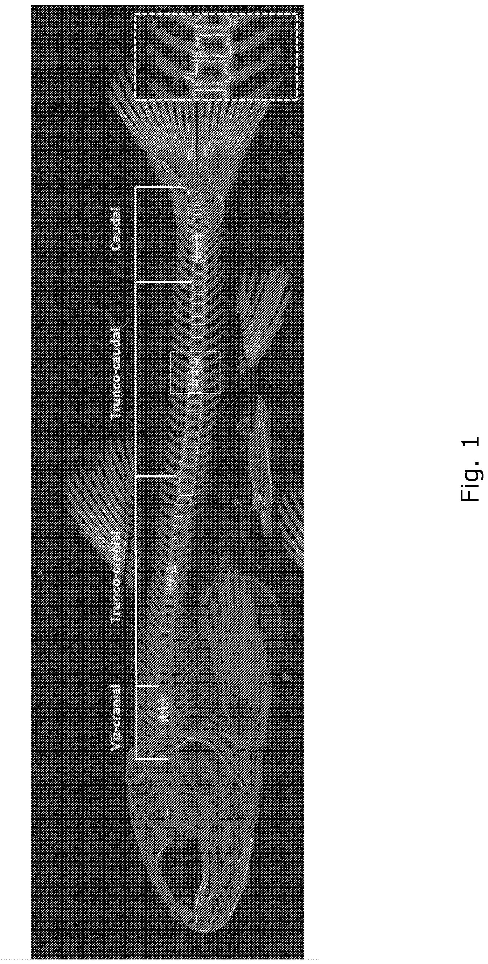

   Figure 1 shows a whole mount double stained specimen (with sharpen edges, ImageJ) showing the four distinct regions considered during measurements of the vertebral column: R1 (viz-cranial, V1→V8), R2 (trunco-cranial, V9→V30), R3 (trunco-caudal, V31→49) and R4 (caudal, V50→60) with R: region and V: vertebrae.
Figure 2:

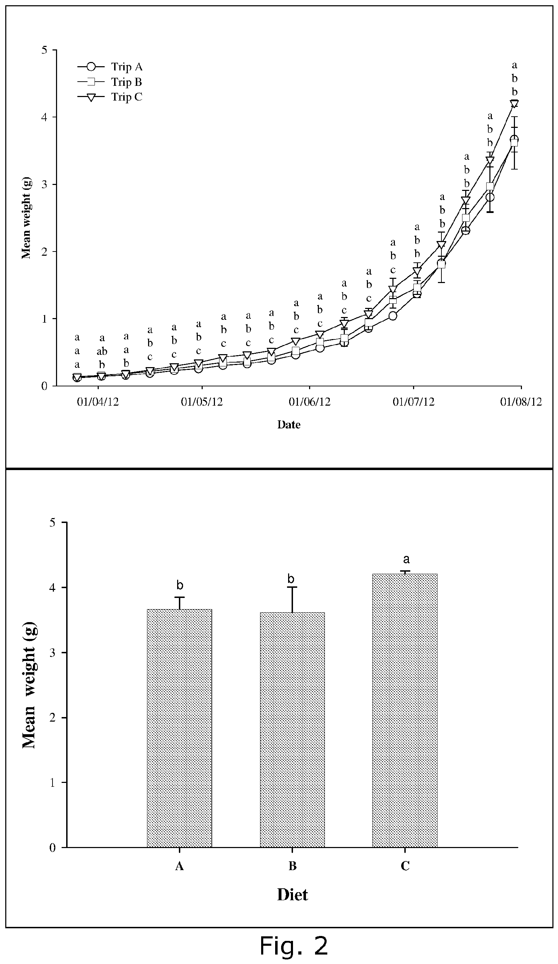

   Figure 2 shows a) Growth performances of triploid Atlantic salmon fed diets at first feeding supplemented with different level of total phosphorus (17, 21 and 23g total phosphorous kg⁻¹) from week 0 to 17 (mean ± S.E.M, n=2). b) Growth performances of triploid Atlantic salmon fed diets at first feeding supplemented with different level of total phosphorus (17, 21 and 23g total phosphorous kg⁻¹) at end of the trial (week 17).
Figure 3:

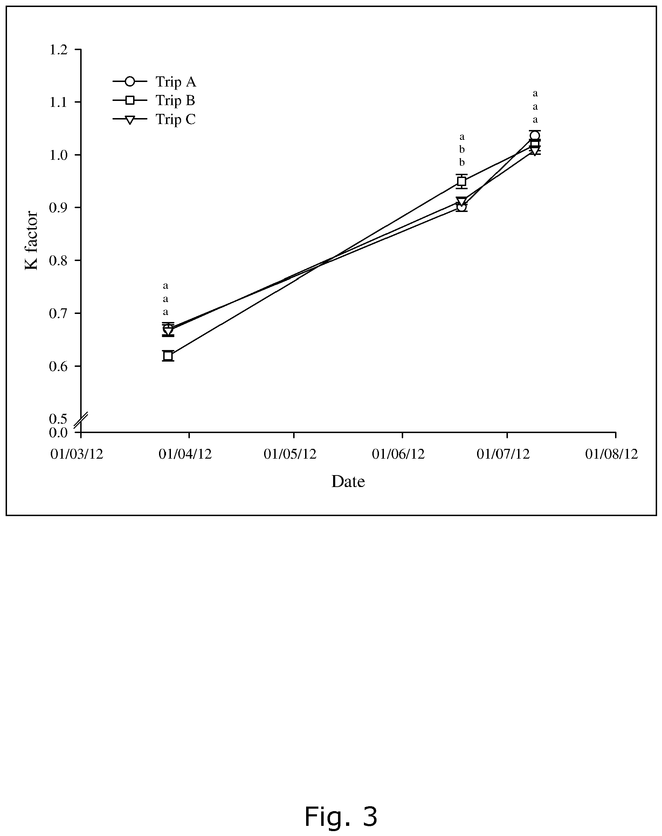

   Figure 3 shows condition factor (K) of triploid Atlantic salmon at first feeding, 1 and 2 g fed diets supplemented with different level of total phosphorus (17, 21 and 23g total phosphorous kg-1).
Figure 4:

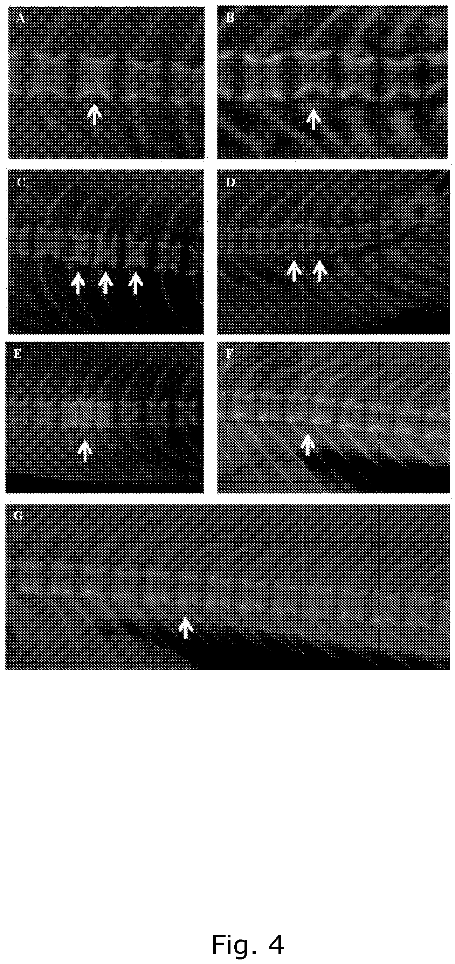

   Figure 4 shows the representative radiographies of triploid Atlantic salmon fry sampled at week 15 post first feeding illustrating some of the most frequent malformations observed in the vertebral column according to Witten *et al.* (2009) classification. A) shows hyper-radiodense vertebrae. B) shows vertically shifted vertebrae. C) shows vertebrae with one sided compression and reduced intervertebral space. D) shows vertically shifted vertebrae with reduced intervertebral space. E) shows compression and reduced intervertebral space. F) shows reduced intervertebral space. F) shows undersized vertebrae. Tables

**Table 1:**

| Table 1 shows the proximate composition (%) of low (A), medium (B) and high phosphorous (C) experimental diets fed to triploid Atlantic salmon during the freshwater phase (mean ± S.E.M, n= 2). | | | |
|---|---|---|---|
| **Proximate composition (%)** | **Diet A Low P** | **Diet B Med P** | **Diet C High P** |
| Protein (%) | 56.5 ± 1.24 | 54.8 ± 0.87 | 54.6 ± 0.79 |
| Lipid (%) | 16.6 ± 0.76 | 17.4 ± 0.66 | 16.6 ± 0.83 |
| Moisture (%) | 6.4 ± 0.55 | 6.8 ± 0.53 | 6.6 ± 0.63 |
| Ash (%) | 7.9 ± 0.26 | 9.0 ± 0.27 | 10.4 ± 0.20 |
| Total P (g kg⁻¹ diet) | 17.14 ± 0.29 | 20.85 ± 0.15 | 24.6 ± 0.75 |

**Table 2**

| Table 2 shows food conversion ratio (FCR)*, specific growth rate (SGR)**, thermal-unit growth coefficient (TGC)*** of triploid Atlantic salmon fed diets supplemented with different level of total phosphorus at first feeding (17, 21 and 23g total phosphorous kg⁻¹ composition) from week 0 to 17 (mean ± S.E.M, n=2). | | | | |
|---|---|---|---|---|
| **Diet** | **Ploidy** | **FCR** | **SGR (% b.w. d-1)** | **TGC (% b.w. oC d-1)** |
| **A** | **Trip** | 1.04 ± 0.24 | 2.74 ± 0.19 | 0.87 ± 0.06 |
| **B** | **Trip** | 0.97 ± 0.16 | 2.61 ± 0.12 | 0.83 ± 0.06 |
| **C** | **Trip** | 0.94 ± 0.01 | 3.06 ± 0.30 | 0.97 ± 0.07 |

| | | | | |
|---|---|---|---|---|
| * Food conversion ratio (FCR) is to be understood as the mass of food consumed divided by the body mass gain, all over a specified time period. ** Specific growth rate (SGR) is defined as an increase in mass of an individual or population over a discrete time period. *** Thermal-unit growth coefficient (TGC), also known as GF3 is to be understood as a defined period of growth or weight gain at a certain temperature over a period of time. | | | | |

**Table 3**

| Table 3 shows Prevalence (%) of external deformities observed in triploid Atlantic salmon at 2g whole body weigth (n=100 fish per replicate/diet), prevalence (%) of vertebral deformities observed by radiology and severity index vertebrae (n=10 fish per replicate/ diet analyzed. ns= not significant, according to a Two Way ANOVA (GLM) followed by Tukey range test. | | | |
|---|---|---|---|
| **Deformities** | **Diet A Low P** | **Diet B Med P** | **Diet C High P** |
| External deformities (%) | 2.0 ± 2.0 | 1.0 ± 1.0 | 0.5 ± 0.5 |
| X-Ray deformities (%) | 90 | 85 | 47.4 |
| Vertebrae number | 59.35 ± 0.11^{ns} | 59.00 ± 0.10^{ns} | 59.11 ± 0.13^{ns} |
| Deformed vertebrae (dV) | | | |
| 0 (dV) % | 10 | 15 | 52.6 |
| 1 (dV) % | 15 | 10 | 10.5 |
| 2-5 (dV) % | 50 | 70 | 36.8 |
| 6-10 (dV) % | 20 | 5 | - |
| >10 (dV) % | 5 | - | - |

**Table 4**

| Table 4 shows Prevalence (%) of observable external deformities in triploid Atlantic salmon at ∼47 g whole body weight (n=50 fish per replicate tank /diet), prevalence (%) of vertebral deformities observed by radiology and severity index vertebrae (n=35 fish per replicate tank/diet analyzed. | | | |
|---|---|---|---|
| **Deformities** | **Diet A Low P** | **Diet B Med P** | **Diet C High P** |
| External deformities (%) | 7.7 ± 0.3 | 2.0 ± 0.0 | 1.2 ± 0.5 |
| X-Ray deformities (%) | 98.6 | 77.1 | 83.6 |
| Vertebrae number | 59.0 ± 0.1 | 59.0 ± 0.1 | 59.0 ± 0.1 |
| Deformed vertebrae (dV) | | | |
| 0 (dV) % | 1.5 | 22.9 | 15.8 |
| 1-5 (dV) % | 55.3 | 64.3 | 73.8 |
| 6-10 (dV) % | 33.1 | 7.1 | 10.4 |
| >10 (dV) % | 10.1 | 5.7 | 0.0 |

### Examples

### Experiment 1

The present study investigated the effects of 3 diets with low (A), medium (B) and high (C) P supplementation, provided from first feeding to ∼4 g whole body weight during the freshwater period, on bone mineralization and development of spinal deformities in triploid Atlantic salmon. Constant monitoring of fish performances, cartilage and bone staining, whole body mineral content and X-ray analyses were conducted to evaluate possible dietary effects on bone mineralization relative to growth rate.

### Materials and methods

### Fish stocks and experimental design

Experiments were conducted at Howietoun Fish Farm and Niall Bromage Freshwater Research Facilities (NBFRF), Stirling. Triploid Atlantic salmon eggs were supplied by AquaGen AS (Norway) and received at Howietoun on the 19/01/12 at approximately 395 degree days (od). Triploid eggs were counted and randomly distributed into three 12 x 250 L square fiberglass tanks (∼5250 eggs/tank) in a flow through system supplied by spring water and supplemental heating until hatch and first feeding (8.7 ± 1.0 °C). Eggs and alevins were maintained under blackout covers until yolk-sac absorption was complete. From first feeding at 929 od post fertilization (26/03/12), duplicate groups were fed one of three experimental diets. The three diets were formulated with graded levels of phosphorous (low (A), medium (B), high (C)) and supplied by BioMar UK Ltd.

Diet A, B and C contained 17, 21 and 23 g total P per kg of diet (∼ 8.5, 10.5, 12.5 g available P per kg of diet) respectively (Table 1). Pellet sizes were gradually increased throughout the trial to fit fish growth (0.5, 0.8, 1.1 and 1.5 mm pellets). Fry were fed according to manufacturer's tabulated guidelines by means of 24 hour clockwork belt feeders. Rations were adjusted on a weekly basis to account for increasing biomass. When fry reach 0.3 g, treatment groups were transferred to NBFRF on-growing unit and distributed into 6 0.9 m3 circular fiberglass tanks supplied at ambient water temperature (11.1 ± 1.7 °C). Fry were raised under constant light (LL) from first feeding throughout duration of the experiment.

### Sample collection

Daily mortalities were recorded for each treatment throughout the whole trial. 100 fish from each treatment were euthanized from first feeding on a weekly basis and bulk weighed (5*20 fish). Fish were euthanized under schedule 1 with a lethal dose of Ms222 anaesthetic. 100 individual weight and length measurements were done on approximately 0.2, 1 and 2 g fry for all treatments and the Fulton's condition factor (K) calculated. Fulton's condition factor (K) is to be understood as a measure of an individual fish's health that uses standard weight, assuming that the standard weight of a fish is proportional to the cube of its length: and is calculated as follows (K) = ((W*100)*1000/(L^3)) where W = weight of individual fish and L= length of the corresponding fish. Five fish from each treatment were sampled at week 0, 1, 3, 8, 12, 15 and fixed in 10% buffered formalin (10:1 v:v) pots prior to whole mount skeletal staining. 10 fish from each treatment were sampled at 1 and 2 g (week 12 and 15) and frozen prior to mineral analyses.

### Whole mount skeletal staining and morphometric analysis

Fry sampled on week 0, 1, 3, 8 and fixed in 10% formalin were double stained with alcian blue and alizarin red S according to Potthoff (1984). Unfortunately week 12 and 15 samples could not be processed due to time constraints. The stained specimens were photographed under a light tent using a Nikon 300S (60 mm F 2.8 microlens) and a light table. Photos were exported to Nikon Capture 2 Pro software on a Windows XP Platform. The number of vertebrae in the vertebral column were assessed on the stained specimens at 0.5 g (n= 5 individuals per replicate, two replicates per treatments) and pooled for analysis. Three vertebrae of the four different anatomical regions in Atlantic salmon described in Kacem et al. (1998), viz-cranial (V1→V8), trunco-cranial (V9→V30), trunco-caudal (V31→49) and caudal (V50→60) were analysed on the stained specimens at 0.5 g (n= 5 individuals per replicate, two replicates per treatments) (Figure 1). The vertebrae counts, length, height, area and intervertebral space of each stained specimen were determined on the digitized photos using image analysis software ImageJ (ImageJ 1.46r, NIH, USA). The degree of ossification was assessed using the mean grey value approach, the mean grey value increasing with bone calcification as described in Fontagné et al. (2009). The mean grey value measurements were conducted on 8-bit converted images.

### Mineral analyses

Mineral compositions were determined on individual whole fish at 1 g (n= 3 individuals per replicate, two replicates per treatments) using nitric digestion standard technique. Fish were oven dried at 105 oC for 15 h, powdered and acid digested using a Mars Microwave digestion system (10 min. heating phase to 160 oC, 20 min. at 160 oC, 30 min. cooling phase). Samples were then analysed via Inductively Coupled Plasma Mass Spectrometry using a Thermo X Series II ICP-MS (collision cell mode). The concentration of minerals present in a sample was calculated as such: M = (sample volume/100) * (result from ICP-MS/sample weight) with M in µg/mg. Nitric/perchloric sample digestion standard technique was used for total phosphorous determination. Total phosphorous was measured using a molybdenum-blue colorimetric procedure with spectrophotometry (ISO 6491-1998), at 690 nm.

### X-ray analyses

X-ray analysis of 2 g fish were conducted using a portable digital specimen radiography system Bioptics BioVision (Daax Ltd, USA). Frozen fish were pooled by 3 or 4 per radiography (calibration: Kv: 22, Time: 15 sec., MAs: 22.52). 10 fish per replicate /diet were analysed. Vertebrae and deformity counts were performed using DICOM analysis software ClearCanvas V.2.0 (ClearCanvas Inc., Toronto, Canada). Vertebral deformities were analysed according to classification of Witten et al. (2009).

### Statistics

All statistical analyses were performed using statistical software Minitab 16.0 (Minitab Inc.) and differences considered significant when P values < 0.05. Differences between dietary treatments were analysed using General Linear Models GLM (Two Way ANOVA) nesting replicate within ploidy and diet. Prior to analyses, datasets were checked for normality according to the Kolmogorov-Smirnov test and for homogeneity using Levene's test. Post-hoc tests were carried out using Tukey's multiple comparisons. Results are presented as mean ± SEM.

### Results

Summation of results indicates that application of a feed with a higher level of phosphorus has the positive effect of reducing the level and severity of deformities in triploid fish that would otherwise be susceptible to development of deformities. Figure 4 and table 3 shows a decrease in both prevalence and severity of the vertebral deformities in the triploid trial fish, with increasing inclusion of phosphorus in the diet. As seen is figure 2 and table 2, there are no detrimental effects on performance or growth from the use of this higher phosphorus diet. Finally, in figure 3, it can be seen that condition factor (K) is on the whole neither positively nor negatively affected by the dietary treatments in the trial. This is a further indication of the success of the trial in terms of the general health and welfare of the trial fish. It is also evidence that the boosted phosphorus levels do not create any negative growth effects, as well as supressing deformities in triploids.

### Experiment 2

The present study investigated the effects of 3 diets with low (A), medium (B) and high (C) P supplementation, provided from first feeding to ∼4 g whole body weight in freshwater, on bone mineralization and development of spinal deformities in triploid Atlantic salmon throughout the freshwater period. X-ray analysis were conducted to evaluate possible dietary effects on bone mineralization.

### Materials and methods

### Fish stocks and experimental design

This study was a continuation of Experiment 1, whereby the duplicate groups of triploid Atlantic salmon continued to be fed with experimental diets with low (A), medium (B) and high (C) P supplementation from 4g whole body weight thoughout the freshweater period to smoltification. At final smoltification, immediately prior to sea water transfer, 35 fish per tank were randomly selected, euthanised with a lethal concentration of anasthetic and stored at -20°C until analysis for x-radiography.

### X-ray analyses

Radiographs were taken of lateral views of smolts from each treatment (35 fish/tank) using a POSKOM PXP-60HF Diagnostic X-ray Unit on an extremities plate (AGFA CRMM 155 3.0 Extremities - code 32) measuring 300x240mm. All plates were exposed and samples processed prior to sample exposure to omit any shadowing caused by natural radiation. Radiographs were exposed with 40kV and 32mAs with 840mm of distance between generator and extremities plate and the image was digitised on site using AGFA CR35-X Digitizer. Images were provided in DICOM format and total vertebrae number and vertebral deformities were analysed using MicroDicom DICOM viewer 0.8.1 (MicroDicom, Sofia, Bulgaria). Vertebral deformities were analyzed according to the classification of Witten et al. (2009).

### Statistics

As described in Experiment 1.

### Results

Prevalence of deformed triploid fish decreased across diets corresponding to increasing P supplementation. Triploid fish fed on diet A had the highest prevalence (98.6%) of deformed individuals and this was reduced in diets B and C (77.1 and 83.6%, respectively, Table 4). The prevalence of moderate or severely deformed individuals decreased with increasing levels of P supplementation (33.1, 7.1, 10.4 % moderate deformed vertebrae for diets A, B and C respectively, and 10.1, 5.7, and 0% severe deformed vertebrae for diets A, B and C respectively, Table 4). These findings would suggest that triploid fish require higher dietary phosphorus to support bone formation and reduce severity of vertebrae deformities compared to diploids.

### References

Asgard, T. & Shearer, K.D. (1997) Dietary phosphorus requirement of juvenile Atlantic salmon, Salmo salar L. Aquaculture Nutrition, 3, 17-23.
Burke, H.A., Sacobie, C.F.D., Laal, S.P., and Benfey, T.J. (2010). The effect of triploidy on juvenile Atlantic salmon (Salmo salar) response to varying levels of dietary phosphorus. Aquaculture, 306, 295-301.
Fontagné, S., Silva, N., Bazin, D., Ramos, A., Aquirre, P., Surget, A., Abrantes, A., 609 Kaushik, S. J. and Power, D. M. (2009). Effects of dietary phosphorus and calcium level on 610 growth and skeletal development in rainbow trout (Oncorhynchus mykiss) fry. Aquaculture 611 297, 141-150.
Kacem, A., Meunier, F. J. and Baglinière, J. L. (1998). A quantitative study of 651 morphological and histological changes in the skeleton of Salmo salar L. during its 652 anadromous migration. Journal of Fish Biology 53, 1096-1109.
Ketola, H.G. & Richmond, M.E. (1994) Requirement of rainbow trout for dietary phosphorus and its relationship to the amount of discharge in hatchery effluent. Trans Am Fish Soc., 123, 597-594.
Potthoff, T. (1984). Clearing and staining techniques. In: Moser, H.G. (Ed.), Ontogeny and 575 Systematics of Fishes. Special Publication-American Society of Ichthyologists and 576 Herpetologists, vol. 1. Allen Press, Lawrence, KS, USA, 35-37.
Witten, P. E., Gil-Martens, L., Huysseune, A., Takle, H. and Hjelde, K. (2009). Towards a 613 classification and an understanding of developmental relationships of vertebral body 614 malformations in Atlantic salmon (Salmo salar L.). Aquaculture 295, 6-14.

## Claims

1. A composition comprising at least 23g total phosphorous per kg composition for use in preventing deformities in triploid fish.

2. The composition according to claim 1, wherein the triploid fish is of the genus *Salmo, Oncorhynchus* and/or *Salvenis.*

3. The composition according to any one of the preceding claims, wherein said composition is a fish feed.

4. The composition according to claim 3, wherein the fish feed is selected from the group consisting of pressed fish feed, agglomerated fish feed, spherized fish feed, extruded fish feed and wet semi moist fish feed.

5. The composition according to any one of claims 3-4, wherein said fish feed comprises conventional feed ingredients selected from the group consisting of a carbohydrate source, a protein source, a lipid source, ash, water and any combinations thereof.

6. The composition according to any one of the preceding claims, wherein the triploid fish of the genus *Salmo* is selected from the group consisting of Atlantic salmon (*Salmo salar*) and Brown trout (*Salmo trutta*).

7. The composition according to any one of the preceding claims, wherein the triploid fish of the genus *Oncorhynchus* is selected from the group consisting of Chinook salmon (*Oncorhynchus tshawytsch*), Rainbow trout (*Oncorhynchus mykiss*), Sockeye salmon (*Oncorhynchus nerka*) and Coho salmon (*Oncorhynchus kisutch).*

8. The composition according to any one of the preceding claims, wherein the triploid fish of the genus *Salvenis* is selected from the group consisting of Arctic charr (*Salvelinus alpinus*), Brook trout (*Salvelinus fontinalis*) and Lake trout (*Salvelinus namaycush*)*.*

9. The composition according to any one of the preceding claims, wherein the phosphorous is selected from the group consisting of Monosodium phosphate (MSP), Monoammonium phosphate (MAP), Monocalcium phosphate (MCP), and phosphorus sources from raw materials.

10. The fish feed according to any one of the preceding claims, wherein the deformity is selected from the group consisting of vertebral deformities, external deformities and jaw deformities.

11. The composition according to any one of the preceding claims, wherein said triploid fish is selected from the group consisting of fry, fingerlings, parr and smolts.

12. The composition according to any one of the preceding claims, wherein said feed is administered to the triploid fish in the fresh water (FW) phase.

13. The composition according to any one of the preceding claims, wherein said feed is administered to the triploid fish from first feeding and until the fish attain a size in the range from 4g-15g whole body weight.

## Patentansprüche

1. Zusammensetzung umfassend insgesamt zumindest 23 g Phosphor pro kg der Zusammensetzung zur Verwendung beim Verhindern von Deformierungen bei triploiden Fischen.

2. Zusammensetzung nach Anspruch 1, wobei die triploiden Fische Fische der Gattung *Salmo, Oncorhynchus* und/oder *Salvenis* sind.

3. Zusammensetzung nach einem der vorstehenden Ansprüche, wobei die Zusammensetzung ein Fischfutter ist.

4. Zusammensetzung nach Anspruch 3, wobei das Fischfutter ausgewählt ist aus der Gruppe bestehend aus gepresstem Fischfutter, agglomeriertem Fischfutter, kugelgeformtem Fischfutter, extrudiertem Fischfutter und nassem, halbfeuchtem Fischfutter.

5. Zusammensetzung nach einem der Ansprüche 3-4, wobei das Fischfutter herkömmliche Futterinhaltsstoffe umfasst, ausgewählt aus der Gruppe bestehend aus einer Kohlenhydratquelle, einer Proteinquelle, einer Lipidquelle, Asche, Wasser und allen Kombinationen davon.

6. Zusammensetzung nach einem der vorstehenden Ansprüche, wobei die triploiden Fische der Gattung *Salmo* aus der Gruppe bestehend aus Atlantischem Lachs (*Salmo salar*) und Bachforelle (*Salmo trutta*) ausgewählt sind.

7. Zusammensetzung nach einem der vorstehenden Ansprüche, wobei die triploiden Fische der Gattung *Oncorhynchus* aus der Gruppe bestehend aus Königslachs (*Oncorhynchus tshawytsch*), Regenbogenforelle (*Oncorhynchus mykiss*), Rotlachs (*Oncorhynchus nerka*) und Silberlachs (*Oncorhynchus kisutch*) ausgewählt sind.

8. Zusammensetzung nach einem der vorstehenden Ansprüche, wobei die triploiden Fische der Gattung *Salvenis* aus der Gruppe bestehend aus Seesaibling (*Salvelinus alpinus*), Bachsaibling (*Salvelinus fontinalis*) und Amerikanischem Seesaibling (*Salvelinus namaycush*) ausgewählt sind.

9. Zusammensetzung nach einem der vorstehenden Ansprüche, wobei das Phosphor aus der Gruppe bestehend aus Mononatriumphosphat (MSP), Monoammoniumphosphat (MAP), Monocalciumphosphat (MCP) und Phosphorquellen aus Rohstoffen ausgewählt ist.

10. Fischfutter nach einem der vorstehenden Ansprüche, wobei die Deformierung aus der Gruppe bestehend aus vertebralen Deformierungen, externen Deformierungen und Kieferdeformierungen ausgewählt ist.

11. Zusammensetzung nach einem der vorstehenden Ansprüche, wobei die triploiden Fische aus der Gruppe bestehend aus Fischbrut, Setzlingen, Parr und Smolt ausgewählt sind.

12. Zusammensetzung nach einem der vorstehenden Ansprüche, wobei das Futter an die triploiden Fische in der Frischwasser- (FW-)Phase verabreicht wird.

13. Zusammensetzung nach einem der vorstehenden Ansprüche, wobei das Futter an die triploiden Fische vom ersten Füttern und bis zu dem Zeitpunkt, zu dem die Fische eine Größe im Bereich von 4 g-15 g Gesamtkörpergewicht erreichen, verabreicht wird.

## Revendications

1. Composition comprenant au moins 23 g au total de phosphore par kg de composition destinée à être utilisée pour la prévention des difformités chez le poisson triploïde.

2. Composition selon la revendication 1, ledit poisson triploïde étant du genre *Salmo, Oncorhynchus* et/ou *Salvenis.*

3. Composition selon l'une quelconque des revendications précédentes, ladite composition étant une nourriture pour poisson.

4. Composition selon la revendication 3, ladite nourriture pour poisson étant choisie dans le groupe constitué par la nourriture pour poisson pressée, la nourriture pour poissons agglomérée, la nourriture pour poisson sphérique, la nourriture pour poisson extrudée et la nourriture pour poisson semi humide mouillée.

5. Composition selon l'une quelconque des revendications 3 et 4, ladite nourriture pour poisson comprenant des ingrédients de nourriture classiques choisis dans le groupe constitué d'une source de glucides, une source de protéines, une source de lipides, de cendre, d'eau et d'une quelconque combinaisons de celles-ci.

6. Composition selon l'une quelconque des revendications précédentes, ledit poisson triploïde du genre *Salmo* étant choisi dans le groupe constitué par le saumon de l'Atlantique (*Salmo salas*) et la truite commune (*Salmo trutta*).

7. Composition selon l'une quelconque des revendications précédentes, ledit poisson triploïde du genre *Oncorhynchus* étant choisi dans le groupe constitué par le saumon chinook (*Oncorhynchus tshawytsch*), la truite arc-en-ciel (*Oncorhynchus mykiss*), le saumon nerka (*Oncorhynchus nerka*) et le saumon coho (*Oncorhynchus kisutch*).

8. Composition selon l'une quelconque des revendications précédentes, ledit poisson triploïde du genre *Salvenis* étant choisi dans le groupe constitué par l'omble chevalier (*Salvelinus alpinus*), l'omble de fontaine (*Salvelinus fontinalis*) et le touladi (*Salvelinus namaycush*)*.*

9. Composition selon l'une quelconque des revendications précédentes, ledit phosphore étant choisi dans le groupe constitué par le phosphate monosodique (MSP), le phosphate de monoammonium (MAP), le phosphate monocalcique (MCP) et des sources de phosphore provenant de matières premières.

10. Nourriture pour poisson selon l'une quelconque des revendications précédentes, ladite difformité étant choisie dans le groupe constitué par les difformités vertébrales, les difformités externes et les difformités de la mâchoire.

11. Composition selon l'une quelconque des revendications précédentes, ledit poisson triploïde étant choisi dans le groupe constitué par les alevins, les fretins, les tacons et les saumoneaux.

12. Composition selon l'une quelconque des revendications précédentes, ladite nourriture étant administrée au poisson triploïde dans la phase d'eau douce (ED).

13. Composition selon l'une quelconque des revendications précédentes, ladite nourriture étant administrée au poisson triploïde depuis la première alimentation et jusqu'à ce que le poisson atteigne une taille comprise dans la plage allant de 4 à 15 g en poids corporel total.
